# EUROPEAN PATENT APPLICATION

(11) **EP 4 014 894 A1**
(43) Date of publication of application: **22.06.2022**
(21) Application number: 20852018.9
(22) Date of filing: 30.07.2020
(51) Int. Cl.: A61B 17/122, A61F 2/24

(54) **ADJUSTABLE VALVE CLAMPING DEVICE AND VALVE CLAMPING SYSTEM**

(30) Priority: 13.08.2019 CN 201910745954; 13.08.2019 CN 201921312186 U
(71) Applicant: Hangzhou Valgen Medtech Co., Ltd., Hangzhou, Zhejiang 310052 (CN)
(72) Inventor: ZHANG, Weiwei, Hangzhou, Zhejiang 310052 (CN); ZHANG, Tingchao, Hangzhou, Zhejiang 310052 (CN); ZHENG, Xianzhang, Hangzhou, Zhejiang 310052 (CN)
(74) Representative: Hamer, Christopher K.
(86) International application number: PCT/CN2020/105950
(87) International publication number: WO 2021/027588

(57) **Abstract**

An adjustable valve clip (100) and a valve clamping system are provided. The valve clamping system includes the adjustable valve clip (100) and a pushing device. The adjustable valve clip (100) includes a pushing rod (40), at least two clip arms (11), and at least one extension arm (20). The pushing rod (40) is configured to axially move to drive the clip arm (11) to be unfolded or folded relative to the pushing rod (40), and drive the extension arm (20) to extend and retract in an axial direction of the clip arm (11). By setting the extendable and retractable extension arm (20) on a surface of the clip arm (11) of the valve clip (100), the extension arm (20) can extends beyond the clip arm (11) when the clip arm (11) is opened relative to the pushing rod (40), which is equivalent to increasing an effective length for gripping the leaflet. The clip arm (11) with a longer length can better support the leaflet when capturing the leaflet, which can prevent the leaflet from slipping off a surface of the clip arm (11). When the leaflet is clamped, the extension arm (20) can be retracted to avoid excessively pulling the leaflet by the clip arm (11) and the extension arm (20) which have an excessively long length. In this way, with aid of an adjustable effective length of clamping, the adjustable valve clip can easily, rapidly, and safely grip the moving leaflet tissue, thereby reducing operation difficulty and improving operation efficiency.

## Description

### TECHNICAL FIELD

This application relates to the field of medical instruments, and in particular to an adjustable valve clip and a valve clamping system.

### BACKGROUND

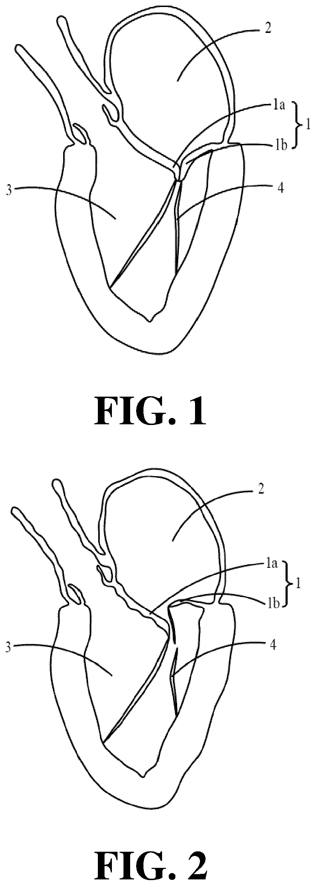

Referring to FIG. 1, a mitral valve 1 is a one-way valve between a left atrium 2 and a left ventricle 3 of the heart. The normal and healthy mitral valve 1 can control blood to flow from the left atrium 2 to the left ventricle 3, while preventing the blood from flowing from the left ventricle 3 to the left atrium 2. The mitral valve has a pair of leaflets, called an anterior leaflet 1a and a posterior leaflet 1b. The anterior leaflet 1a and the posterior leaflet 1b are fixed on an inner wall of the left ventricle 3 through a chordae tendineae 4. Under normal circumstances, during systole of the left ventricle of the heart, edges of the anterior leaflet 1a and the posterior leaflet 1b are in full coaptation to prevent the blood from flowing from the left ventricle 3 to the left atrium 2. Referring to FIG. 2, when the mitral valve leaflets or associated structures thereof have organic or functional lesion (for example, partial rupture of the chordae tendineae 4), insufficient coaptation of the anterior leaflet 1a and the posterior leaflet 1b of the mitral valve 1 may be caused. During systole of the left ventricle of the heart, the mitral valve 1 cannot be closed sufficiently, so that the blood flows back from the left ventricle 3 to the left atrium 2, thereby causing a series of pathological and physiological changes, which are referred to as "mitral valve regurgitation".

At present, there is a minimally invasive surgery, in which a leaflet clamp is delivered to the mitral valve through a delivery device, and then the anterior and posterior leaflets of the mitral valve are simultaneously clamped by relatively opening and closing the clamp, so that the anterior and posterior leaflets of the mitral valve are fixed, thereby reducing the mitral valve regurgitation. However, since the two leaflets of the mitral valve are kept in a state of large-range and intensive opening and closing movement, it is more difficult for the clamp to quickly and successfully grip the moving leaflet tissue, and operation time is longer. However, if the difficulty of gripping is reduced by directly increasing the size of the clamp, the difficulty of delivering the clamp may be increased, or clamping arms may pull the leaflets excessively to damage the leaflets.

### SUMMARY

An objective of this application is to provide an adjustable valve clip and a valve clamping system in view of the above-mentioned defects of the conventional art. The adjustable valve clip can easily, rapidly, and safely grip the moving leaflet tissue, thereby reducing operation difficulty and improving operation efficiency.

The adjustable valve clip includes a pushing rod, a fixing base, at least two clip arms, and at least one extension arm. The pushing rod extends through the fixing base and is axially movable relative to the fixing base. The clip arm has a clamping section and a driving section connected with the clamping section. The clip arm is hinged to the fixing base at a position between the clamping section and the driving section. The driving section is connected with the pushing rod, and the pushing rod is configured to axially move to drive the clip arm to rotate about the position where the clip arm is hinged to the fixing base, to be unfolded or folded relative to the pushing rod. The extension arm is operable to extend and retract in an axial direction of the clip arm. When the extension arm extends in a direction from the driving section to the clamping section of the clip arm, a tail end of the extension arm extends beyond an end of the extension arm away from the driving section.

The valve clamping system includes a pushing device and the above adjustable valve clip. The pushing device includes an operating handle and a pushing shaft having an axial length. A proximal end of the pushing shaft is connected to the operating handle. A distal end of the pushing shaft is detachably connected to the adjustable valve clip.

The adjustable valve clip and the valve clamping system are provided. By setting the extension arm to be able to extend or retract in the axial direction of the clip arm, a distal end of the extension arm extends beyond an end of the clip arm away from the driving section when the clip arms are opened relative to the pushing rod, which is equivalent to increasing an effective length for gripping the leaflet. The clip arm with a longer length can better support the leaflet when capturing the leaflet, which can prevent the leaflet from slipping off a surface of the clip arm. When the leaflet is clamped, the extension arm can be retracted to avoid excessively pulling the leaflet by the clip arm and the extension arm which have an excessively long length. In this way, with aid of an adjustable effective length of clamping, the adjustable valve clip can easily, rapidly, and safely grip the moving leaflet tissue, thereby reducing operation difficulty and improving operation efficiency.

### BRIEF DESCRIPTION OF THE DRAWINGS

To clearly describe structural characteristics and functions of this application, this application will be described in detail below with reference to the accompany drawings and specific embodiments.
FIG. 1 is a schematic diagram of a mitral valve in a normal state.
FIG. 2 is a schematic diagram of a mitral valve with a lesion.
FIG. 3 is a schematic structural view of an adjustable valve clip in an opening state according to an embodiment of this application.
FIG. 4 is a front view showing opening of clip arms of the adjustable valve clip shown in FIG. 3.
FIG. 5 is a front view showing closing of the clip arms of the adjustable valve clip shown in FIG. 3.
FIG. 6 is a location diagram of an adjustable valve clip, at the mitral valve, of this application.
FIG. 7 is a schematic diagram of the mitral valve during systole after leaflets are clamped by an adjustable valve clip of this application.
FIG. 8 is a schematic diagram of the mitral valve during diastole after leaflets are clamped by an adjustable valve clip of this application.
FIG. 9 is a schematic structural view of a connection between a pushing rod and a base of the adjustable valve clip shown in FIG. 3.
FIG. 10 is a schematic structural view of a connection between a base connecting tube and a fixing base of the adjustable valve clip shown in FIG. 3.
FIG. 11 is a sectional view of a connection structure of the base connecting tube and the fixing base of the adjustable valve clip shown in FIG. 10.
FIG. 12 is a schematic structural view of a clip arm of the adjustable valve clip shown in FIG. 3.
FIG. 13 is a schematic structural view of a clip arm of an adjustable valve clip according to another embodiment of this application.
FIG. 14 is a schematic structural view of an adjustable valve clip according to another embodiment of this application.
FIG. 15 is a schematic structural view showing closing of clamping arms of the adjustable valve clip shown in FIG. 3.
FIG. 16 is a schematic structural view showing opening of the clamping arms of the adjustable valve clip shown in FIG. 3.
FIG. 17 is a schematic structural view of an adjustable valve clip in a closing state according to another embodiment of this application.
FIG. 18 is a schematic structural view of an extension arm of the adjustable valve clip according to the embodiments shown in FIG. 3 and FIG. 17.
FIG. 19 is a sectional view of an adjustable valve clip in an opening state according to another embodiment of this application.
FIG. 20 to FIG. 24 are schematic structural views of bearing parts of extension arms of adjustable valve clips according to different embodiments of this application.
FIG. 25 is a schematic structural view of an adjustable valve clip in an opening state when a bearing part of an extension arm is a structure according to an embodiment shown in FIG. 24.
FIG. 26 and FIG. 27 are schematic structural views of bearing parts of extension arms of adjustable valve clips according to two other different embodiments of this application.
FIG. 28 is a schematic structural view of a connection between an adjustable valve clip and a pushing shaft when clip arms of the valve clip according to an embodiment of this application are in an opening state.
FIG. 29 is a sectional view of the connection between the adjustable valve clip and the pushing shaft shown in FIG. 28.
FIG. 30 is a schematic structural view of a connection between an adjustable valve clip and an adjustable pushing shaft when clip arms of the adjustable valve clip according to an embodiment of this application are in a closing state.
FIG. 31 is a sectional view of a connection between the adjustable valve clip and the pushing shaft shown in FIG. 30.

### DETAILED DESCRIPTION

The technical solutions in the embodiments of this application are clearly and completely described below in conjunction with the accompanying drawings of this application. The accompanying drawings are only used for exemplary description, which are only schematic diagrams, and cannot be understood as a limitation of this application. It needs to be emphasized that in this application, "proximal end" indicates a direction close to an operator, and "distal end" indicates a direction away from the operator.

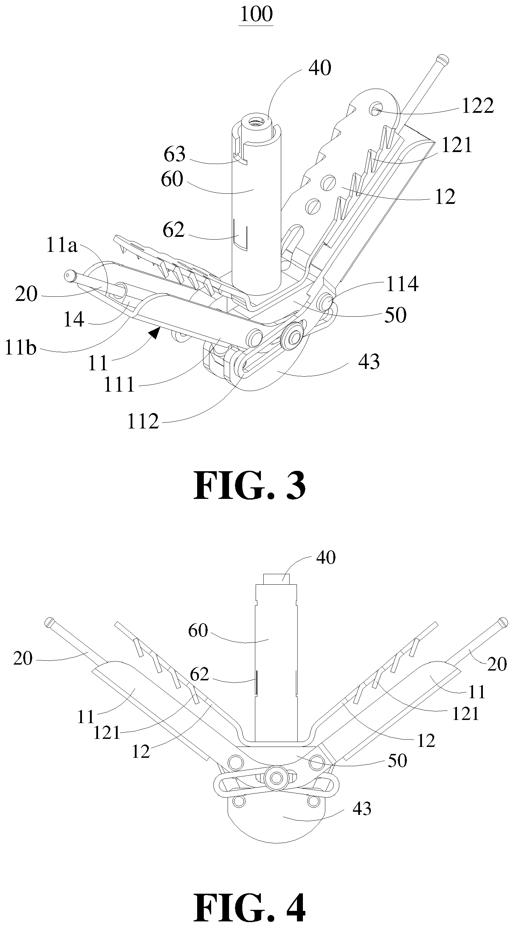

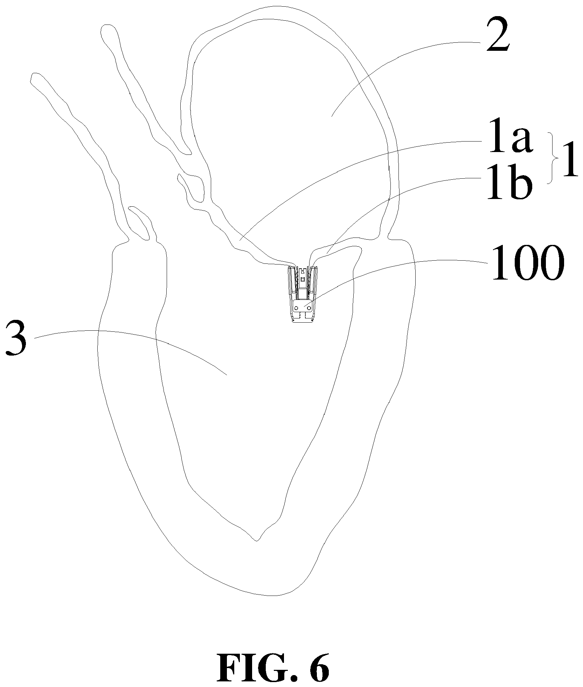

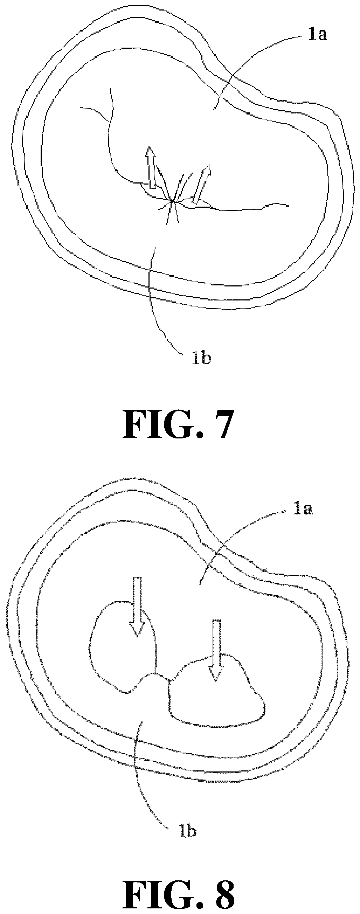

Referring to FIG. 3 to FIG. 5, an adjustable valve clip 100 is provided in this application. The adjustable valve clip 100 includes a pushing rod 40, a fixing base 50, at least two clip arms 11, and at least one extension arm 20. In this embodiment, the number of the clip arms 11 is two. The two clip arms 11 are arranged symmetrically around the pushing rod 40 and can be unfolded or folded relative to the pushing rod 40. The valve clip 100 of this application can be used for edge-to-edge repair to prevent mitral valve regurgitation. Specifically, referring to FIG. 6, the valve clip 100 is placed at a position where the anterior leaflet and the posterior leaflet of the mitral valve cannot achieve coaptation properly, such that one clip arm 11 supports the edge of the anterior leaflet 1a of the mitral valve, and the other clip arm 11 supports the edge of the posterior leaflet 1b of the mitral valve. Then the anterior leaflet 1a and the posterior leaflet 1b are respectively fixed through cooperation between the clip arms 11 and the pushing rod 40, so that the anterior leaflet 1a and the posterior leaflet 1b of the mitral valve are fixed together at the position where they cannot achieve coaptation properly. FIG. 7 is a diagram showing a state of the mitral valve during systole, where an arrow direction indicates a direction of blood flow. During systole, the anterior leaflet 1a and the posterior leaflet 1b are closed together, and the edge of the anterior leaflet 1a and the edge of the posterior leaflet 1b are clamped by the valve clip 100, so that the mitral valve can be completely closed or an opening area thereof is reduced, thereby achieving alleviation or treatment of "mitral valve regurgitation". Referring to FIG. 8, FIG. 8 shows a state of the mitral valve during diastole, where an arrow direction indicates a direction of blood flow. During diastole, the anterior leaflet 1a and the posterior leaflet 1b are fixed together only at a position where the valve clip 100 clamps the anterior leaflet 1a and the posterior leaflet 1b, and the other positions of the anterior leaflet 1a and the posterior leaflet 1b are still in normal dilatation. As such, a lot of blood can enter the left ventricle from the left atrium, thereby ensuring normal circulation of the blood. In other embodiments of this application, the adjustable valve clip 100 can also be used to alleviate or treat "tricuspid valve regurgitation". That is, the adjustable valve clip 100 has three clip arms 11, and three leaflets are clamped with the three clip arms 11, so that the "tricuspid valve regurgitation" can be alleviated or avoided. The principle and structure are the same as those of the adjustable valve clip 100 for resolving the mitral valve regurgitation in the embodiments of this application, which will not be elaborated here. It can be understood that, in other embodiments of this application, the adjustable valve clip 100 may also be applied to other minimally invasive surgical operations where several pieces of tissue need to be clamped together, and the number of clip arms 11 can be changed according to actual usage requirements.

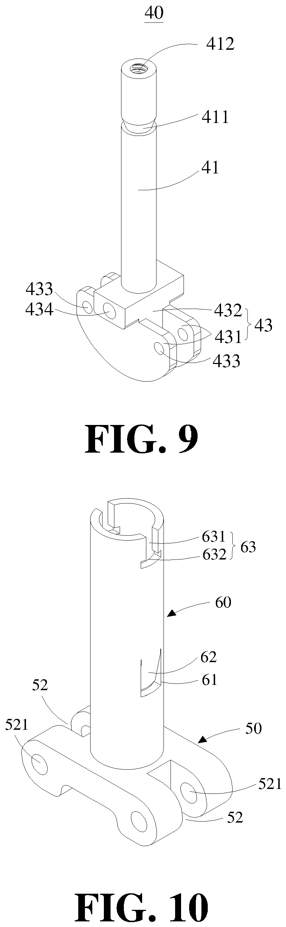

Referring to FIG. 3 and FIG. 9 together, the pushing rod 40 extends through the fixing base 50 and is axially movable relative to the fixing base 50, so as to push the clip arms 11 to be unfolded or folded relative to the pushing rod 40 and drive the extension arm 20 to move. In this way, a distal end of the extension arm 20 can extend beyond or retract to one end of the clip arm 11. A proximal end of the pushing rod 40 circularly defines a catching groove 411. A threaded hole 412 is defined in a direction from an end face of the proximal end of the pushing rod 40 to the inside of the pushing rod 40, and is used to connect with a delivery system of the adjustable valve clip 100. The pushing rod 40 includes a pushing rod body 41 and a connecting base 43 disposed at a distal end of the pushing rod body 41. The pushing rod body 41 may be of a rod-like structure with a cross section in any shape. For example, the pushing rod body 41 may be a round rod or a square rod. In this embodiment, the pushing rod body 41 is a round rod, so that the pushing rod 40 can be moved more smoothly.

In some embodiments of this application, the connecting base 43 includes two fixing plates 431 that are opposite to each other and a fixing block 432 fixed on the two fixing plates 431. The fixing block 432 is partially located between the two fixing plates 431 to connect the two fixing plates 431 and is partially disposed on end faces of the two fixing plates 431 facing the pushing rod 40. Two opposite ends of each fixing plate 431 define pin holes 433, and the pin holes 433 in the two fixing plates 431 are defined opposite to each other. The fixing block 432 is disposed on the end faces of the two fixing plates 431 facing the pushing rod 40 and also defines a pin hole 434. The pin holes 433 and the pin hole 434 have the same axial directions. The connecting base 43 has a smooth outer surface, so that the connecting base 43 can be smoothly pushed and can be prevented from damaging the human tissue or hooking the chordae tendineae. The connecting base 43 may be a structure in any shape, for example, a cuboid structure, a hemispheroid structure, a spherical crown structure, or a bullet-shaped structure. Preferably, a size of a cross section of the connecting base 43, where the cross section is perpendicular to an axial direction of the pushing rod 40, is gradually decreased from a proximal end to a distal end, so that the adjustable valve clip 100 can be pushed in the human body more easily.

The pushing rod 40 and the connecting base 43 are made of a biocompatible metal material which is specifically selected from at least one of stainless steel, cobalt-chromium alloy, cobalt alloy, or titanium alloy, preferably titanium alloy.

Referring to FIG. 3, FIG. 10, and FIG. 11, in some embodiments of this application, the adjustable valve clip 100 further includes a base connecting tube 60. The base connecting tube 60 is sleeved on the pushing rod 40 and fixed with the fixing base 50. The fixing base 50 defines a through hole 51. The through hole 51 is connected with a tubular cavity of the base connecting tube 60. The pushing rod 40 movably extends through the base connecting tube 60 and passes through the through hole 51 of the fixing base 50. The pushing rod 40 can axially move relative to the base connecting tube 60 and the fixing base 50. In some embodiments, the fixing base 50 defines grooves 52 at two opposite sides of the fixing base 50. Pin holes 521 are respectively defined on opposite groove walls of each groove 52. An axial direction of each pin hole 521 is perpendicular to that of the pushing rod 40.

The base connecting tube 60 defines an opening 61. A resilient piece 62 is disposed in the opening 61. The resilient piece 62 includes a connecting end and a free end opposite to the connecting end. The connecting end is connected to the edge of the opening 61. In a natural state, the free end inclines to the inside of the base connecting tube 60 relative to the connecting end. When the clip arms 11 are closed to the pushing rod 40, the free end of the resilient piece 62 is engaged in the catching groove 411 at the proximal end of the pushing rod 40, so that the pushing rod 40 is prevented from moving axially and the clip arms 11 are prevented from being opened relative to the pushing rod 40.

Furthermore, a connecting portion 63 is disposed at a proximal end of the base connecting tube 60 and used to connect with a pushing device that pushes the adjustable valve clip 100 to the cardiac valve. In some embodiments of this application, the connecting portion 63 is a T-shaped slot, and the T-shaped slot includes a first slot section 631 and a second slot section 632 intersected with the first slot section 631. An extension direction of the first slot section 631 is the same as an axial direction of the base connecting tube 60, and the first slot section 631 extends from an end face of a proximal end of the base connecting tube 60 toward a distal end of the base connecting tube 60.

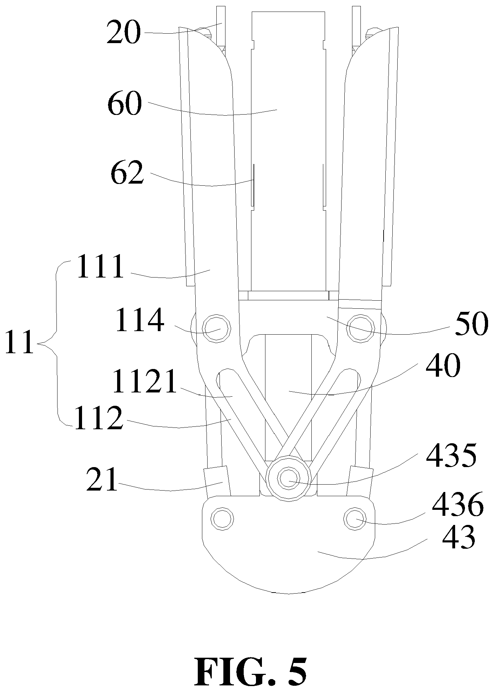

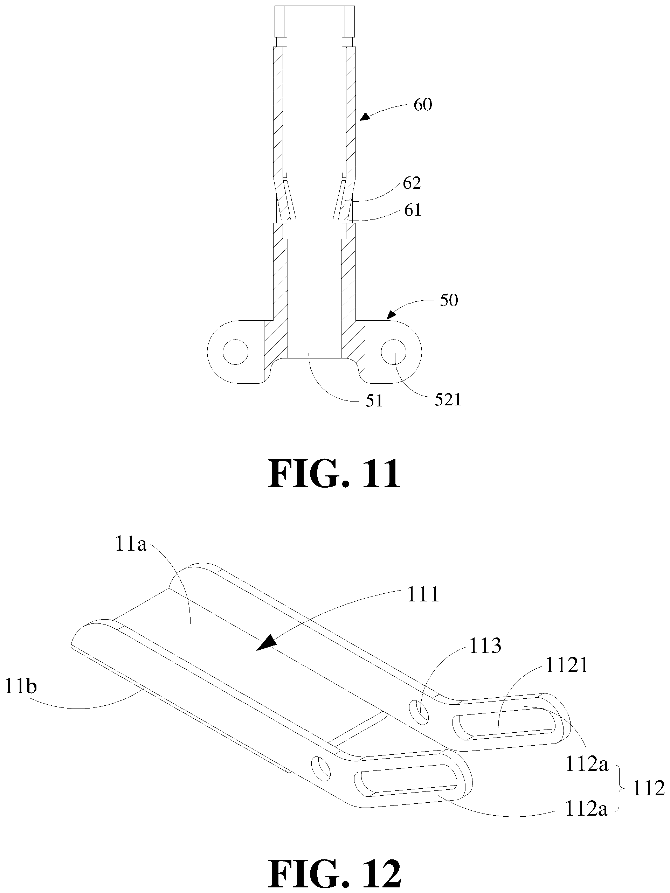

Referring to FIG. 5 and FIG. 12 together, FIG. 12 is a schematic structural view of a clip arm 11 in some embodiments of this application. Each clip arm 11 has a clamping section 111 and a driving section 112 connected with the clamping section 111. The clip arms 11 are hinged to the fixing base 50 at a position between the clamping section 111 and the driving section 112. The clamping section 111 is of a plate-like structure. The driving section 112 includes two connecting bars 112a disposed in parallel. The two connecting bars 112a are connected to a proximal end of the clamping section 111. In some embodiments, the clamping section 111 and the driving section 112 are molded integrally to obtain an integrated structure. In the embodiment, an end face of the clamping section 111 facing the driving section 112 is in contact with an end face of the driving section 112 facing the clamping section 111, so as to achieve a connection between the driving section 112 and the clamping section 111. Hinge holes 113 are defined at a position where the clamping section 111 is hinged with the driving section 112. A pin 114 extends through the pin holes 521 in the fixing base 50 and the hinge holes 113 to hinge the clip arm 11 to the fixing base 50, such that the pushing rod 40 can axially move to drive the clip arms 11 to be rotate about the position (i.e., the hinge holes 113) where the clip arm 11 is hinged to the fixing base 50, and to be opened or closed relative to the pushing rod 40.

The driving section 112 defines slide slots 1121 extending in a length direction of the driving section 112. Specifically, the slide slots 1121 are respectively defined on the connecting bars 112a of the driving section 112. The driving section 112 is connected with the pushing rod 40. The pushing rod 40 has limiting posts fixed at a distal end of the pushing rod, where the limiting rods protrude from a surface of the pushing rod 40 and are perpendicular to the axial direction of the pushing rod 40, and extend through the slide slots 1121 of the driving section 112. The pushing rod 40 is configured to axially move to drive the limiting posts to slide in the slide slots 1121, so as to drive the clip arms 11 to rotate about the position where the clip arm 11 is fixed to the fixing base 50 to be unfolded or folded relative to the pushing rod 40. In this embodiment, a pin 435 extends through the pin hole 434 of the fixing block 432 of the connecting base 43, and two ends of the pin 435 expose to the pin hole to protrude from the surface of the connecting base 43. That is, parts, exposed to the pin holes, of two ends of the pin 435 are the limiting posts. The two connecting bars 112a of the driving section 112 are respectively located on two sides of the fixing block. The parts (the limiting posts), exposed to the pin holes 434, of the pin 435 respectively pass through the slide slots 1121 on the two connecting bars 112a, and can move along the slide slots 1121, so that when the pushing rod 40 drives the connecting base 43 to axially move, the limiting posts can slide in the slide slots 1121, such that the clip arms 11 can be driven to rotate about the position (i.e., the hinge holes 113) where the clip arm 11 is hinged to the fixing base 50, to be opened or closed relative to the pushing rod 40. It can be understood that in some embodiments, alternatively, the limiting posts may be of protrusion structures fixed on two opposite surfaces of the connecting base 43.

The clamping section 111 includes a first surface 11a facing the pushing rod 40. The first surface 11a is a recessed surface that recesses in a direction facing away from the pushing rod 40, so that the clip arms 11 can be closed to the pushing rod 40 more tightly. In some embodiments, the recessed surface is a cambered surface, and a radius of curvature of the cambered surface is slightly greater than that of the pushing rod 40.

Furthermore, in some embodiments, the clamping section 111 of the clip arm 11 may be applied with an active drug, or may define one or multiple through holes 115 arranged at intervals, which not only assists in crawling and growth of endothelial cells but also can reduce weight of the clip arm 11.

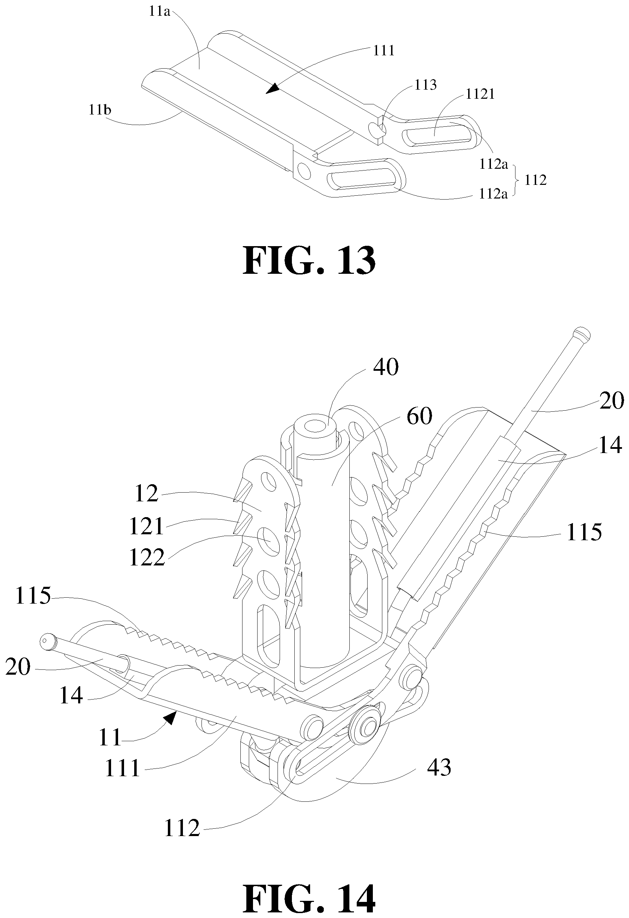

Referring to FIG. 13, FIG. 13 shows a clip arm 11 in another embodiment of this application. The difference from the clip arm 11 shown in FIG. 12 lies in that a clamping section 111 and a driving section 112 are connected with each other by lap jointing, and hinge holes 113 are defined at lap joints of the clamping section 111 and the driving section 112.

Referring to FIG. 14, in some other embodiments of this application, the clip arm 11 defines a friction enhancement structure 115 to enhance a friction force between the clip arm 11 and the leaflets, thereby providing a stable clamping force. The friction enhancement structure 115 may be protrusions or slots that are disposed on the first surface 11a of the clip arm 11 facing the pushing rod 40, or may be a pad that fits the first surface 11a and is made of a biocompatible material with a high friction coefficient. In an embodiment, the friction enhancement structure 115 is a sawtooth structure arranged at the edges of the clip arm 11. In order to ensure a stable clamping force and to adapt to the size of the leaflets, the clamping sections 111 of the clip arms 11 have a certain size specification. Specifically, when the clamping sections 111 of the clip arms 11 are too long, the clamping sections 111 of the clip arms 11 may excessively clamp the anterior leaflets 1a and the posterior leaflets 1b, and the two leaflets are forcedly pulled to each other and fixed together, which easily results in dysfunction of the mitral valve. In addition, during heart beating and leaflet movement, too many parts of the leaflets are restricted from movement, which may lead to a severe result such as leaflet tear. When the clamping sections 111 of the clip arms 11 are too short, only a small part of the leaflets can be clamped, making the leaflets easy to slip off and resulting in a poor clamping effect. In some embodiments of this application, an axial length (that is, a length in a direction from the driving section 112 to the clamping section 111) of the clamping section 111 of each clip arm 11 may be greater than or equal to 4 mm, preferably 6-10 mm. A width of the clamping section 111 of the clip arm 11 is also limited to a certain extent, so that impact on the clamping effect resulted with too small width of the clamping section 111 of the clip arm 11 and impact on the leaflet movement resulted with too large width of the clamping section 111 of the clip arm 11 are avoided. The width (i.e., a length in a direction perpendicular to an axial direction of the clamping section 111 of the clip arm 11) of the clamping section 111 of the clip arm 11 may be greater than or equal to 2 mm, preferably 4-6 mm. In order to ensure safety after implantation, the clip arm 11 may be made of a biocompatible material and have certain flexibility and rigidity, so as to avoid damage to the leaflets and tightly clamp the leaflets. Specifically, the biocompatible material is selected from stainless steel, cobalt alloy, cobalt-chromium alloy, or titanium alloy.

Referring to FIG. 3 to FIG. 5, in an embodiment of this application, the adjustable valve clip 100 includes a clamping component with elasticity, and the clamping component includes two clamping arms 12 disposed at an angle. Each clamping arm 12 corresponds to one clip arm 11 and is located between the clip arm 11 corresponding thereto and the pushing rod 40. Each clip arm 11 and one clamping arm 12 are closed to clamp the leaflet.

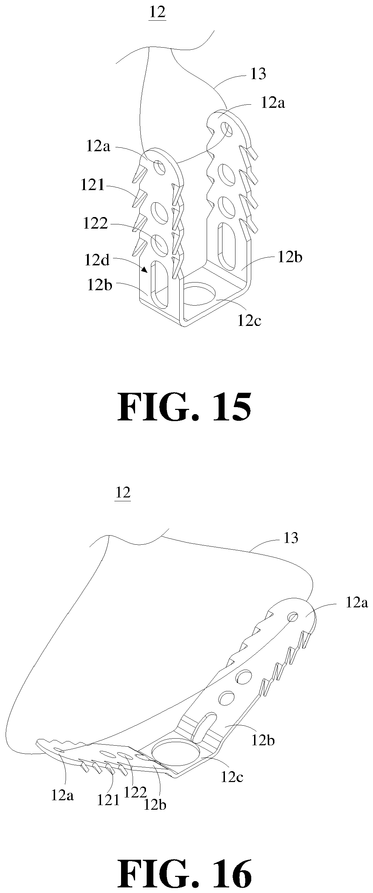

Referring to FIG. 3, FIG 15, and FIG. 16, FIG. 15 is a schematic structural view of a clamping component in a closing state (i.e., a delivery state) according to some embodiments of this application, and FIG. 16 is a schematic structural view of a clamping component in a natural state (i.e., a released state) according to some embodiments of this application. The clamping component is U-shaped, and the shape shown in FIG. 16 is obtained through cutting an elastic material with a shape memory function, such as nickel-titanium alloy, and heat setting treatment. In this application, the shape memory function refers to a function that deformation is generated when an external force is applied and the original shape can be recovered after the external force disappeared. Due to the elasticity and the shape memory function, the clamping component can be delivered in a catheter when in the state shown in FIG. 15 and can be returned to the state shown in FIG. 16 after being released from restriction, thereby clamping the leaflets by matching with the clip arms 11. Each clamping arm 12 of the clamping component includes a free end 12a and a fixing end 12b disposed opposite to each other. The fixing end 12b is fixed on the fixing base 50. In some embodiments, the fixing end 12b is fixed between the fixing base 50 and the base connecting tube 60. In some embodiments, the fixing ends 12b of the two clamping arms 12 are connected with a connecting plate 12c to form an integrated structure, and the connecting plate 12c and the fixing base 50 are in a detachable or a non-detachable connection, so that the fixing ends 12b of the two clamping arms 12 and the fixing base 50 are fixed. In some embodiments of this application, at least parts of the clamping arms 12 are made of an elastic material such as nickel-titanium alloy. In an embodiment of this application, the fixing ends 12b of the clamping arms 12 are made of an elastic material, and the free ends 12a of the clamping arms 12 may be made of a non-elastic material such as aluminum alloy, so that the free ends 12a are driven to move through resilience of the fixing ends 12b. In the natural state, each clamping arm 12 is disposed at an angle relative to the pushing rod 40. An included angle between axial directions of the two clamping arms 12 is in a range of 0 degree to 160 degrees. In some embodiments, the included angle between the two clamping arms 12 may be slightly greater than an included angle between the two clip arms 11 so that a more stable clamping force is provided. That is, for each clamping arm 12, the included angle between the clamping arm 12 and the pushing rod 40 is greater than or equal to an included angle between the clip arm 11 corresponding to the clamping arm 12 and the pushing rod 40 when the clip arm 11 corresponding to the clamping arm 12 is opened to the utmost extent, so that a certain clamping force between the clip arm 11 and the clamping arm 12 is ensured to clamp the leaflets between the clip arm 11 and the clamping arm 12.

The free ends 12a of the clamping arms 12 are connected to a control part 13 in the pushing device. The control part 13 is configured to control the free ends 12a of the clamping arms 12, so that opening and closing between the clamping arms 12 and the pushing rod 40 as well as the clip arms 11 are adjusted. In this embodiment, the control part 13 is an adjusting wire made of metal or a polymer material such as polytetrafluoroethylene (PTFE). The adjusting wire extends through the free ends 12a of the clamping arms 12 to restrict the clamping arms 12 onto the surface of the pushing rod 40, so that the two clamping arms 12 are kept in a closing state, which is conductive to delivery by a sheath through a curved blood vessel. After positions between the clip arms 11 and the leaflets are adjusted well, the free ends 12a of the clamping arms 12 are released from control of the adjusting wire. Due to the elastic memory properties, the clamping arms 12 recover to two sides and move closer from the pushing rod 40 to the clip arms 11. At this point, the clamping arms 12 are in a natural state and matched with the clip arms 11 to respectively clamp the leaflets between the clamping arms 12 and the clip arms 11.

Furthermore, each clamping arm 12 has a third surface 12d facing the clip arm 11 and a clamping reinforcer 121 on the third surface 12d, so as to increase a friction force between the clamping arm 12 and the leaflet and increase a clamping force bwtween the clip arm 11 and the clamping arm 12 to the leaflet. The clamping reinforcer 121 may be a structure such as ribs, barbs, bosses, or other irregularly distributed protrusions arranged on the third surface 12d protrusively, and alternatively, may be a rough surface at least partially covering the third surface 12d. For example, the third surface 12d of the clamping arm 12 is covered with a pad made of a biocompatible material with a higher friction coefficient, so that the third surface 12d has an increased surface roughness coefficient, thereby increasing a clamping force of the adjustable valve clip 100 to the leaflets. Alternatively, the clamping reinforcer 121 may be a magnetic body disposed on the clamping arm 12. Here, the clip arm 11 is also provided with a magnetic body correspondingly. With a mutual magnetic attraction force between the clip arm 11 and the clamping arm 12, the purpose of enhancing the clamping force is achieved.

In an embodiment of this application, the clamping reinforcer 121 refers to two rows of teeth disposed at intervals. The two rows of teeth are disposed opposite at edges of two side of the clamping arm 12. An included angle between an axial direction of each tooth and the third surface 12d is less than or equal to 90 degrees, thereby further enhancing the clamping force. Furthermore, an end of each tooth away from the third surface 12d is a smooth curved surface, so as to avoid damage to the leaflet. In this embodiment, the clamping arm 12 defines a through hole 122, so that the clamping arm 12 has a reduced weight and an improved elasticity, and crawling and growth of the endothelial cells are facilitated. Furthermore, in some embodiments, since a width of the clip arm 11 is less than that of the clamping arm 12, when the clip arm 11 and the clamping arm 12 are closed, the teeth on the clamping arm 12 are located at two sides of the clip arm 11, and the first surface of the clip arm 11 touches the third surface 12d of the clamping arm 12, so that impact from the teeth to the closing of the clip arm 11 and the clamping arm 12 is avoided.

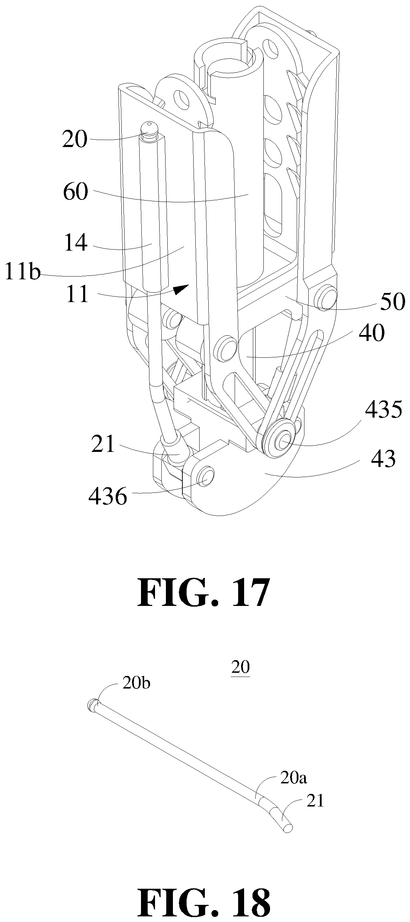

Referring back to FIG. 3, and FIG. 17 to FIG. 19, at least one extension arm 20 is provided. The extension arm 20 is disposed on the surface of or inside the clip arm 11. Preferably, at least one extension arm 20 is disposed on the inner surface and/or the outer surface of each clip arm 11, thus each clip arm 11 may have an increased length and can grip the leaflets more easily. In an embodiment of this application, two extension arms 20 are respectively disposed on the surfaces of the two clip arms 11. Specifically, each extension arm 20 may be disposed on the first surface 11a (that is, the inner surface of the clip arm 11) of each clip arm 11 or disposed on the second surface 11b (that is, the outer surface of the clip arm 11) facing away from the first surface 11a. In the embodiment shown in FIG. 3, the extension arm 20 is disposed on the first surface 11a of the clip arm 11. Specifically, the first surface 11a of the clip arm 11 is provided with a limiting part 14. The limiting part 14 is used to limit that an extension direction of the extension arm 20 is the direction from the driving section 112 of the clip arm 11 to the clamping section 111, thereby restricting a radial deflection of the extension arm 20. The limiting part 14 may be a limiting ring, a limiting slot, a limiting tube, or other various limiting structures. Specifically, in this embodiment, the limiting part 14 is a tube with a certain length. The extension arm 20 may movably extends through the tube. In an embodiment shown in FIG. 17, the extension arm 20 is disposed on the second surface 11b of the clip arm 11. Specifically, the second surface 11b of the clip arm 11 is provided with the limiting part 14, and the extension arm 20 extends through the limiting part 14 to extend or retract along the direction from the driving section 112 to the clamping section 111 of the clip arm 11. In some embodiments, the extension arm 20 is disposed inside the clip arm 11. In an embodiment shown in FIG. 16, the clip arm 11 defines a through hole extending from the driving section 112 to the clamping section 111, and the extension arm 20 penetrates the through hole, so as to dispose the extension arm 20 inside the clip arm 11.

Referring to FIG. 18, in some embodiments of this application, the extension arm 20 has a fixing end 20a and a free end 20b opposite to the fixing end 20a, and the fixing end 20a is rotationally fixed at the distal end of the pushing rod 40. In an embodiment of this application, the fixing end 20a is hinged to the connecting base 43 at the distal end of the pushing rod 40, and specifically located between the two fixing plates 431 on the connecting base 43. The fixing end 20a defines a pin hole. A pin 436 extends through the pin hole and the two opposite pin holes 433 of the two fixing plates 431, so that the fixing end 20a is hinged to the connecting base 43, and the extension arm 20 can be rotationally fixed relative to the connecting base 43. When the pushing rod 40 is axially pushed, the pushing rod 40 drives a tail end of the extension arm 20 to extend beyond or retract to an end of the clip arm 11 away from the driving section 112. Specifically, when axially moving to the distal end, the pushing rod 40 can drive the tail end of the extension arm 20 to retract in the axial direction of the clip arm 11, or when axially moving to the proximal end, the pushing rod 40 can drive the tail end of the extension arm 20 to extend in the axial direction of the clip arm 11. At this time, in some embodiments of this application, since the axial movement of the pushing rod 40 can drive the clip arms 11 to be opened or closed relative to the pushing rod 40 and drive the extension arms 20 to extend or retract in the axial directions of the clip arms 11, extension or retraction of the extension arms 20 is synchronized with the unfolding or folding of the clip arms 11 relative to the pushing rod 40. Therefore, without adding of extra operation steps, the effective length of the clip arms 11 can be increased by controlling the extension arms 20 to extend or retract relative to the axial directions of the clip arms 11, thereby reducing operation difficulty.

In some embodiments, the adjustable valve clip 100 further includes an extension arm steel sleeve 21. The fixing end 20a of the extension arm 20 is fixed on the extension arm steel sleeve 21 through welding or crimping, or the like. An end of the extension arm steel sleeve 21 away from the extension arm 20 is located between the two fixing plates 431 on the connecting base 43 and defines a pin hole. A pin extends through the pin hole on the extension arm steel sleeve 21, and two ends of the pin are fixed in the two opposite pin holes 433 of the two fixing plates 431, thus rotational fixing between the extension arm steel sleeve 21 and the connecting base 43 is achieved, thereby achieving rotational fixing between the fixed end 20a of the extension arm 20 and the connecting base 43.

In this embodiment, when the pushing rod 40 is pushed to the distal end, the limiting posts are moved inside the slide slots 1121 to the clamping section 111. At this point, a distance from the limiting posts to the tail end (i.e., an end of the clamping section 111 away from the driving section 112) of the clamping section 111 is reduced, so that the tail end of the extension arm 20 can partially extend beyond the end of the clip arm 11 away from the driving section 112. When the pushing rod 40 is pulled to the proximal end, the limiting posts are moved inside the slide slots 1121 to a direction away from the clamping section 111. At this point, the distance from the limiting posts to the tail end (i.e., the end of the clamping section 111 away from the driving section 112) of the clamping section 111 is increased, so that the tail end of the extension arm 20 can retract to the end of the clip arm 11 away from the driving section 112. In some embodiments, the length of the extension arm 20 is the same as that of the clip arm 11. When the clip arm 11 is opened relative to the pushing rod 40, the distance from the limiting posts to the tail end (that is, the end of the clamping section 111 away from the driving section 112) of the clamping section 111 is reduced, that is, a length of the part of the clip arm 11 clamping the leaflet is reduced. However, after a length of the extension arm 20 extending out of the tail end of the clip arm 11 is added, the actual length of the clip arm 11 gripping the leaflet does not change, thus the clip arm 11 still can grip the leaflet relatively easily. However, since the distance from the limiting posts to the tail end of the clamping section 111 is reduced, the length of the clip arm 11 clamping the leaflet is reduced, thereby avoiding problems that the leaflets are pulled excessively due to an excessive length of the adjustable clip valve 100, or the like.

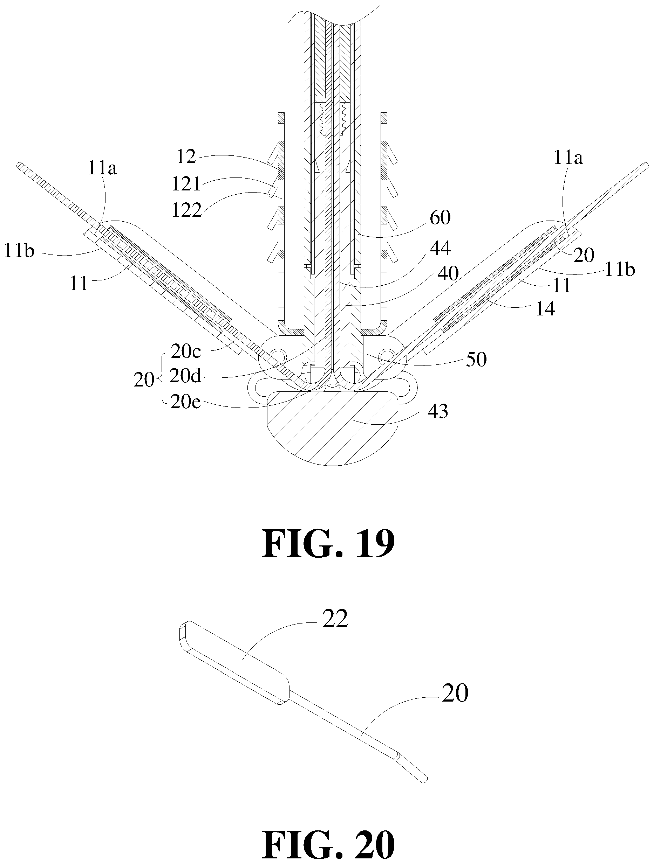

Referring to FIG. 19, in other embodiments of this application, extension or retraction of the extension arm 20 may be unsynchronized with the unfolding or folding of the clip arms 11 relative to the pushing rod 40. In the embodiment shown in FIG. 19, the pushing rod 40 defines a hollow cavity 44, and the hollow cavity 44 extends in the same direction as the axial direction of the pushing rod 40. The extension arm 20 is made of an elastic material. The extension arm 20 has a first section 20c, a second section 20d, and a third section 20e that is connected with the first section 20c and the second section 20d. The third section 20e is a curved section. The first section 20c can extend or retract in the axial direction of the clip arm 11. The second section 20d movably extends through the hollow cavity 44. The second section 20d can further extend to an outside of the body of the patient or be connected to other control bar extending outside the body of the patient. When moving along the hollow cavity 44, the second section 20d can drive the first section 20c to extend or retract in the axial direction of the clip arm 11. In this embodiment, with the axial movement of the pushing rod 40, the clip arm 11 is opened fully, and then the second section 20d of the extension arm 20 is driven to move along the hollow cavity 44, so that a tail end of the first section 20c of the extension arm 20 extends out of the end of the clip arm 11 away from the driving section 112, and the tail end of the first section 20c of the extension arm 20 can be adjusted in real time to extend to a proper position. Furthermore, after the clip arms 11 are closed relative to the pushing rod 40, the extension arms 20 can be removed out of the body, thus implanted components are reduced.

Preferably, the overall extension arm 20 is smooth, an end extending out of the clip arm 11 is provided with a smooth round head formed by laser spot welding, and the smooth round head has no defects such as burrs, edges, and corners, avoiding damage to the leaflets.

In some embodiments of this application, the extension arm 20 includes an extension arm body. The extension arm body includes one or more supporting rods arranged side by side, and the supporting rods are used to directly support the leaflets. The supporting rods may be of a solid or hollow structure and may also be of a single-layer or multi-layer composite structure. Furthermore, in this embodiment, the supporting rods are made of a flexible and/or elastic biocompatible material to be adaptable to an anatomical structure of the leaflets and a range of movement of the leaflets, thereby avoiding damage to the leaflets. For example, a metal material, a polymer material, or a metal-polymer composite material can be selected for preparation. Specifically, the supporting rods are made of a metal-polymer composite material. In this embodiment, the supporting rods are made of nickel-titanium alloy and PTFE.

Furthermore, in other embodiments of this application, the extension arm 20 includes an extension arm body made of a flexible material and further includes a supporter made of a rigid material such as, stainless steel, titanium alloy, or the like. The supporter is disposed inside and/or outside the extension arm body to increase a strength of the extension arm 20, so that the extension arm 20 has a certain flexibility to be adaptable to the anatomical structure of the leaflets and the range of movement of the leaflets and also has a certain rigidity to effectively support the leaflets. For example, in another embodiment of this application, the extension arm body is formed by winding at least one flexible wire (e.g., a stainless steel wire). The extension arm body is covered with a thermoplastic elastomer (e.g., pebax), and heating is performed to melt the pebax to cover the extension arm body. In the meantime, the pebax may partially permeate the inside of the extension arm body via gaps among the flexible wire. Therefore, at this time, the supporter is disposed both inside and outside the extension arm body. Furthermore, at least part of the extension arm 20 is made of a radiopaque material. For example, the extension arm body and/or the supporter is made of the radiopaque material, so that the extension arm 20 may swing correspondingly along with the movement of the leaflet after extending out of the adjustable valve clip 100 to touch the leaflet, then the position of the leaflet can be rapidly and accurately determined by an operator through X-ray. If the position is reasonable, the adjustable valve clip 100 can be driven by the operator to clamp the leaflets, so that operation time is reduced and a success rate of operation is increased. The radiopaque material is selected from stainless steel or nickel-titanium alloy.

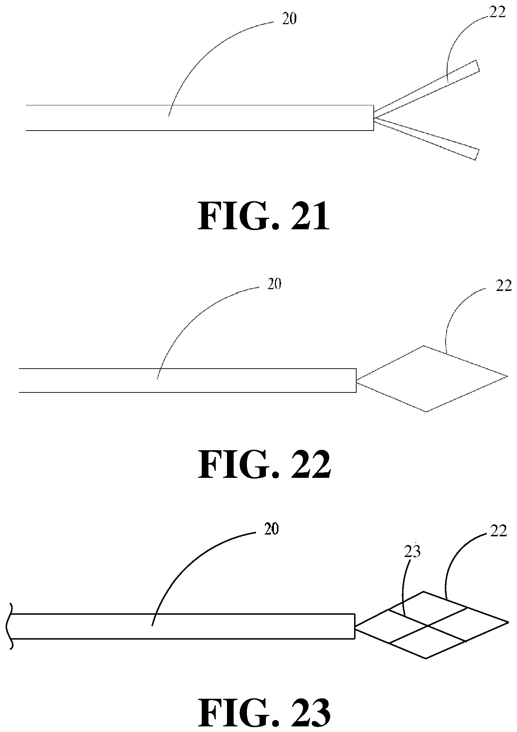

Referring to FIG. 20 to FIG. 24, FIG. 20 to FIG. 24 are schematic structural views of extension arms according to some other embodiments of this application. In these embodiments, the free end 20b of the extension arm 20 is further provided with a bearing part 22. When the tail end of the extension arm 20 extends beyond an end of the clip arm 11 away from the driving section 112, a width of the bearing part 22 is greater than a diameter of the extension arm 20, so that the bearing part 22 has a larger supporting area for bearing the leaflets better. A width direction of the bearing part 22 is the same as a width direction of the clip arm 11. The bearing part 22 may be of a plate-like structure with or without elasticity and may also be other elastic members. As shown in FIG. 20, the bearing plate 22 is of a plate-like structure, and particularly a flat plate without elasticity. As shown in FIG. 21 to FIG. 24, the bearing part 22 is an elastic member. The elastic member is compressed and kept in a compressed state when retracting to the clip arm 11 along with the extension arm 20. When the elastic member extends out of the clip arm 11 along with the extension arm 20, i.e., when the tail end of the extension arm 20 extends beyond the end of the clip arm 11 away from the driving section 112, the elastic member is released from an external force and kept in a released state. The area of the elastic member in the released state is greater than that of the elastic member in the compressed state, thus the elastic member has a larger contact area with the leaflet, thereby fitting the leaflet better and improving the supporting effect of the extension arm 20 to the leaflet. Preferably, in this application, a plane where the released elastic member is located is parallel to the first surface 11a of the clip arm 11, thus a larger contact area is achieved between the released elastic member and the leaflet, thereby achieving a better supporting effect to the leaflet. Specifically, both the first surface 11a and the plane where the released elastic member is located may be flat surfaces or curved surfaces.

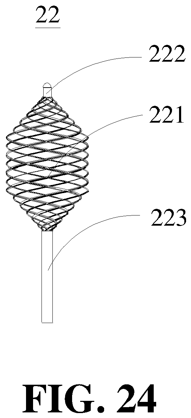

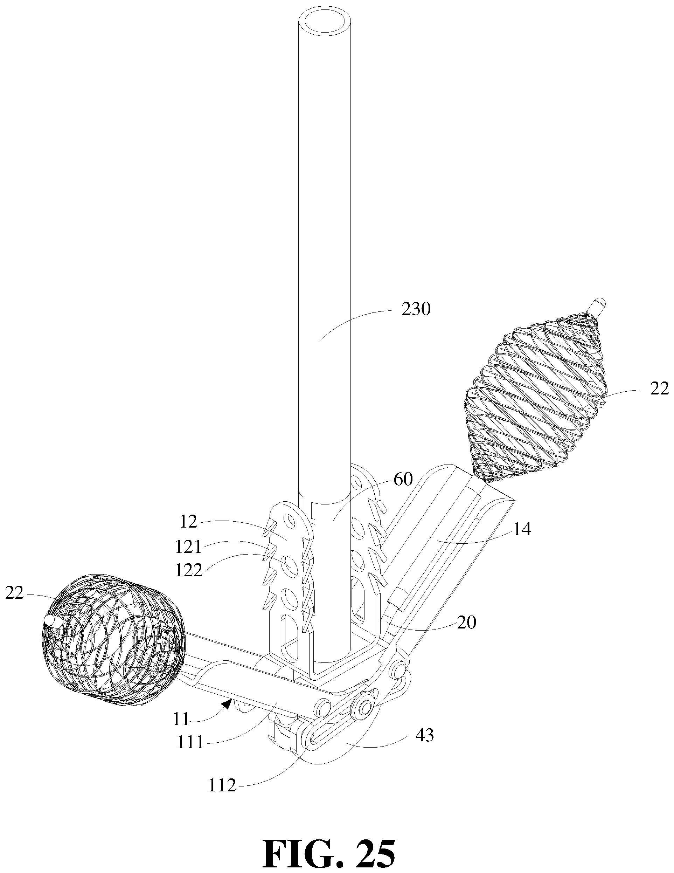

In an embodiment shown in FIG. 21, the bearing part 22 includes multiple branches. When the bearing part 22 is retracted to the clip arm 11, the multiple branches are bunched together. When the bearing part 22 extends out of the clip arm 11, the multiple branches stretch, so that the area of the bearing part 22 is expanded relative to the bearing part 22 retracted to the clip arm 11. In an embodiment shown in FIG. 22, the bearing part 22 is a closed elastic ring. The elastic ring may be of a closed structure in a circular shape, a diamond shape, an oval shape, a pear shape, a polygon shape, or other irregular shapes. When the elastic ring is retracted to the clip arm 11, the elastic ring is squeezed and deformed. When the elastic ring extends out of the clip arm 11, the elastic ring is released, and the area of the released elastic ring is greater than that of the squeezed elastic ring, so that the supporting effect of the extension arm 20 to the leaflet is improved. Furthermore, referring to FIG. 23, at least one supporting bar 23 is disposed in the elastic ring, to improve stability of the elastic ring. Furthermore, an extension direction of the supporting bar is the same as the direction from the driving section 112 of the clip arm 11 to the clamping section 111, thus the elastic ring still can easily extend or retract in the clip arm 11 after the supporting bar 51 is added to the elastic ring. Referring to FIG. 24, in another embodiment of this application, the bearing part 22 is a deformable mesh cage. Specifically, the bearing part 22 is of a mesh cage structure woven by threads with certain elasticity and tension. When the mesh cage is retracted into the clip arm 11, the mesh cage is squeezed and deformed to be in a compressed state. When the mesh cage extends out of the clip arm 11, the mesh cage is released, and the volume of the released mesh cage is greater than that of the squeezed mesh cage, so that more stable support is provided for the leaflets supported on the extension arm 20. In this application, the thread used to form the mesh cage may be an elastic metal wire or polymer wire with the shape memory function. In this embodiment, a super-elastic nickel-titanium wire is used to form the mesh cage, where the super-elastic nickel-titanium wire has relatively good biocompatibility and can be developed under X-rays. Furthermore, compared with the bearing part 22 in the embodiments shown in FIG 20 to FIG. 23, the bearing part 22 in this embodiment has a three-dimensional structure, so that the bearing part 22 can achieve a more three-dimensional development effect and provide more stable support for the leaflets supported on the extension arm 20 (as shown in FIG. 25).

Referring to FIG. 24 and FIG. 25 together, in this embodiment, the mesh cage has a woven net 221, an end socket 222, and a fixing tube 223, where the end socket 222 and the fixing tube 223 are respectively fixed to both ends of the woven net 221. Specifically, the cylindrical woven net 221 is formed with the nickel-titanium wire, and one end of the woven net 221 is fixed in the end socket 222. That is, an opening end of the woven net 221 is closed and fixed by the end socket 222. The other end of the woven net 221 is closed and fixed in the fixing tube 223. An end of the fixing tube 223 away from the woven net 221 is connected to the extension arm 20. Both the end socket 222 and the fixing tube 223 may be made of a metal material or a polymer material. In this embodiment, both the end socket 222 and the fixing tube 223 are made of stainless steel.

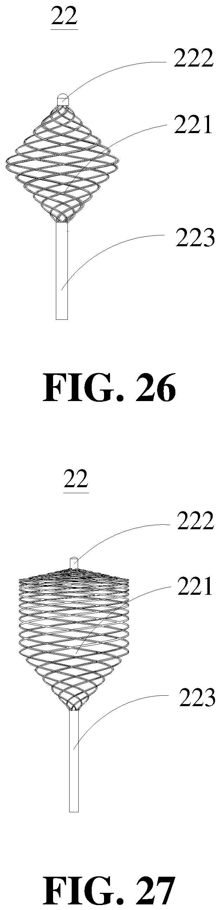

In this embodiment, the mesh cage has a columnar at the middle part and two conical ends with the same cone angles. It can be understood that in this application, the mesh cage may also be in other shapes. For example, referring to FIG. 26 and FIG. 27, the mesh cage may be of a spindle-shaped structure with the same cone angles at two ends as shown in FIG. 26 or a structure with different cone angles at the two ends as shown in FIG. 27.

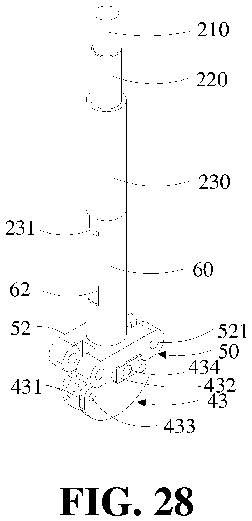

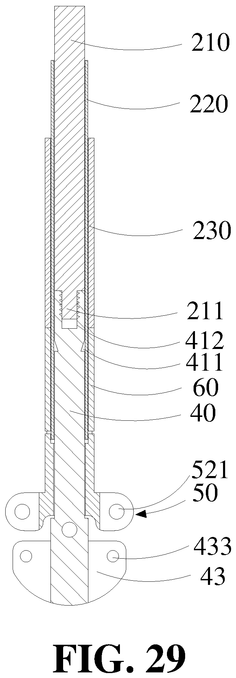

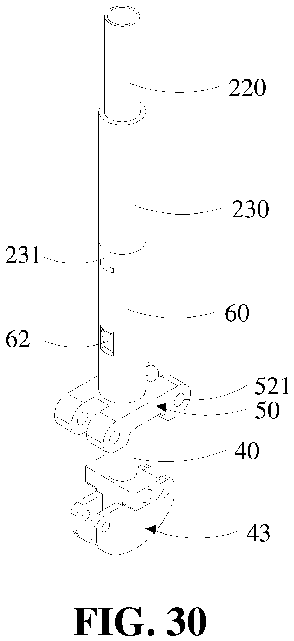

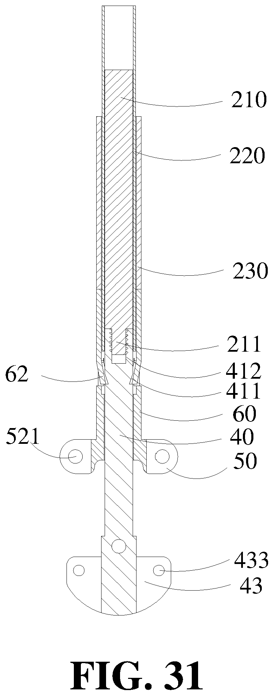

Referring to FIG. 28 to FIG. 31, a valve clamping system is further provided in this application and includes a pushing device and the adjustable valve clip 100 described above. Through the pushing device, the adjustable valve clip 100 may be delivered to the mitral valve and adjusted to a proper position of the mitral valve. The pushing device includes an operating handle for an operator to hold and a pushing shaft that is connected to a distal end of the operating handle and has a certain axial length. A proximal end of the pushing shaft is connected to the operating handle, and the distal end of the pushing shaft is detachably connected to the adjustable valve clip 100. Specifically, the pushing shaft includes a mandrel 210, a bushing 220 sleeved on the mandrel 210, and an outer tube 230 sleeved on the bushing 220. The operating handle can respectively drive the mandrel 210, the bushing 220, and the outer tube 230 to move relatively.

A distal end of the mandrel 210 is provided with an external thread 211 corresponding to an internal thread in the threaded hole 412 at the proximal end of the pushing rod 40. When the pushing shaft is connected to the adjustable valve clip 100, the distal end of the mandrel 210 and the proximal end of the pushing rod 40 are in threaded connection. The operating handle drives the mandrel 210 to move, so as to drive the pushing rod 40 to move axially. In some embodiments, the mandrel 210 is a hollow tube. The second section 20d of the extension arm 20 extends through the mandrel 210 to connect with the operating handle, so that a length of the tail end of the extension arm 20 extended beyond the end of the clip arm 11 away from the driving section 112 can be adjusted outside the human body by means of the operating handle.

A T-shaped resilient piece 231 is disposed at a distal end of the outer tube 230 and used to be matched with a T-shaped slot 63 on the base connecting tube 60, to achieve connection and disconnection between the outer tube 230 and the base connecting tube 60. In a natural state, one end of the T-shaped resilient piece is connected to the distal end of the outer tube 230, while the other end thereof inclines to the axis of the outer tube 230. Specifically, when the pushing shaft is connected to the valve clip 100, the mandrel 210 and the pushing rod 40 are in threaded connection, and the operating handle drives the bushing 220 to move. As such, when the bushing 220 is inserted into the base connecting tube 60, the bushing 220 lifts the T-shaped resilient piece 231 of the outer tube 230, so that the T-shaped resilient piece 231 is embedded in the T-shaped slot 63 of the base connecting tube 60. At this point, the base connecting tube 60 and the outer tube 230 are connected. When the operating handle is operated to drive the bushing 220 to leave the base connecting tube 60, the T-shaped resilient piece 231 of the outer tube 230 is in a natural state, i.e., the T-shaped resilient piece is deformed inwards and separated from the T-shaped slot 63, therefore the base connecting tube 60 and the outer tube 230 are unlocked.

The following takes a mitral valve repair process as an example to illustrate an operation method of the valve clamping system of this application, which mainly includes the following steps.

At the first step, the pushing shaft is connected to the adjustable valve clip 100. The mandrel 210 of the pushing shaft is rotated to be fixed to the pushing rod 40. The bushing 220 is axially moved to the distal end, so that the T-shaped resilient piece 231 of the outer tube 230 is lifted and then embedded into the T-shaped slot 63 of the base connecting tube 60, thereby keeping the base connecting tube 60 and the outer tube 230 in a connection state. At this point, the free end of the resilient piece 62 on the base connecting tube 60 is located in the circular groove 411 of the pushing rod 40, so that both the clip arm 11 and the clamping arm 12 are closed to the surface of the pushing rod 40. At this time, the connection state between the pushing shaft and the adjustable valve clip 100 is as shown in FIG. 21 and FIG. 22.

At the second step, through the pushing shaft, the adjustable valve clip 100 connected thereto is pushed from the left atrium to enter the left ventricle through the mitral valve.

At the third step, the bushing 220 is axially moved to the distal end, to lift the free end of the resilient piece 62 on the base connecting tube 60, so that the free end of the resilient piece 62 is separated from the circular groove 411. At this point, the pushing rod 40 can axially move in the base connecting tube 60.

At the fourth step, the mandrel 210 is moved to the proximal end through the operating handle, to drive the pushing rod 40 connected to the mandrel 210 to move to the proximal end, thereby driving the clip arms 11 to be opened or closed relative to the pushing rod 40 and driving the extension arms 20 to extend out of the clip arms 11.

It can be understood that when the adjustable valve clip 100 is the valve clip shown in FIG. 19, the second section 20d of the extension arm 20 can be pushed to the distal end by means of the operating handle, so that the second section 20d pushes the distal end of the first section 20c to move further away from the end of the clip arm 11 away from the driving section 112, thereby further prolonging the actual length of the clip arm 11 gripping the leaflet.

At the fifth step, the direction of the adjustable valve clip 100 is adjusted, and the relative position of each clip arm 11 with the anterior leaflet 1a and the posterior leaflet 1b is observed through X-rays and other equipments, thereby making the clip arm 11 perpendicular to a coaptation line of the mitral valve.

At the sixth step, the entire adjustable valve clip 100 is retracted to the proximal end, so that the clip arms 11 and the extension arms 20 can support the leaflets at a side of the left ventricle.

At the seventh step, the clamping arms 12 are released by means of the control part, so that the anterior leaflet 1a and the posterior leaflet 1b of the mitral valve are respectively clamped between a pair of clip arms 11 and clamping arms 12, thereby gripping the leaflets.

At the eighth step, the mandrel 210 is moved to the distal end to drive the pushing rod 40 to move to the distal end, thereby driving the clip arms 11 to be closed and retracting the extension arms 20 into the limiting parts 14, that is, retracting the tail ends of the extension arms 20.

At the ninth step, the outer tube 230 is fixed, the bushing 220 is withdraw by a certain distance to the proximal end. Here, the free end of the resilient piece 62 of the base connecting tube 60 is engaged in the circular groove 411 of the pushing rod 40, to ensure that the clip arms 11 are always closed. The mandrel 210 is controlled to rotate through the operating handle, so that the thread between the mandrel 210 and the pushing rod 40 is disconnected. The bushing 220 and the mandrel 210 are withdrawn towards the proximal end until the T-shaped resilient piece 231 of the outer tube 230 is unlocked and separated from the T-shaped catching groove 63 of the base connecting tube 60. At this point, the adjustable valve clip 100 is completely separated from the pushing shaft. The pushing shaft is withdrawn from the body of the patient, and the valve clip 100 remains in the body of the patient to complete the edge-to-edge repair of the mitral valve leaflets.

The valve clamping system of this application can be operated outside the body to clamp the valve leaflets with the valve clip, thereby alleviating or avoiding the "mitral valve regurgitation". Moreover, since the valve clip can easily grip the leaflets, the difficulty of the "mitral valve regurgitation" operation through the valve clamping system is greatly reduced, and the operation time is saved.

The above is only the specific embodiment manner of the present application and not intended to limit the scope of protection of the present application. Any variations or replacements apparent to those skilled in the art within the technical scope disclosed by the present application shall fall within the scope of protection of the present application. Therefore, the scope of protection of the present application shall be subject to the scope of protection of the claims.

## Claims

1. An adjustable valve clip, comprising:
a pushing rod;
a fixing base, wherein the pushing rod extends through the fixing base and is axially movable relative to the fixing base;
at least two clip arms, wherein the clip arm has a clamping section and a driving section connected with the clamping section, the clip arm is hinged to the fixing base at a position between the clamping section and the driving section, the driving section is connected with the pushing rod, and the pushing rod is configured to axially move to drive the clip arm to rotate about the position where the clip arm is hinged to the fixing base, to be unfolded or folded relative to the pushing rod; and
at least one extension arm, wherein the extension arm is operable to extend and retract in an axial direction of the clip arm, and when the extension arm extends in a direction from the driving section to the clamping section of the clip arm, a tail end of the extension arm extends beyond an end of the extension arm away from the driving section.

2. The adjustable valve clip of claim 1, wherein
the driving section defines slide slots extending in a length direction of the driving section;
the pushing rod has limiting posts fixed at a distal end of the pushing rod, wherein the limiting posts protrude from a surface of the pushing rod and are perpendicular to an axial direction of the pushing rod, and extend through the slide slots of the driving section; and
the pushing rod is configured to axially move to drive the limiting posts to slide in the slide slots, so as to drive the clip arms to rotate about the position where the clip arm is fixed to the fixing base to be unfolded or folded relative to the pushing rod.

3. The adjustable valve clip of claim 1, wherein the clip arm defines a friction enhancement structure, and the friction enhancement structure is used to enhance a friction force between the clip arm and a leaflet.

4. The adjustable valve clip of claim 1, wherein the extension arm movably extends on a surface of the clip arm or inside the clip arm.

5. The adjustable valve clip of any of claims 1-4, wherein
the extension arm has a fixing end and a free end opposite to the fixing end, the fixing end is hinged to a distal end of the pushing rod; and
the pushing rod is configured to axially move to drive the clip arms to be unfolded or folded relative to the pushing rod and to drive the extension arm to extend or retract in the axial direction of the clip arm.

6. The adjustable valve clip of claim 5, wherein
extension or retraction of the extension arms is synchronized with unfolding or folding of the clip arms relative to the pushing rod; or
the extension or retraction of the extension arms is unsynchronized with the unfolding or folding of the clip arms relative to the pushing rod.

7. The adjustable valve clip of any of claims 1-4, wherein
the pushing rod defines a hollow cavity, the hollow cavity extending in the same direction as an axial direction of the pushing rod; and
the extension arm has a first section, a second section, and a third section that is connected with the first section and the second section, wherein the third section is a curved section, the second section movably extends through the hollow cavity, and the second section is operable to move along the hollow cavity to drive the first section to extend or retract in the axial direction of the clip arm.

8. The adjustable valve clip of claim 1, wherein
the tail end of the extension arm is further provided with a bearing part; and
when the tail end of the extension arm extends beyond an end of the clip arm away from the driving section, a width of the bearing part is greater than a diameter of the extension arm, and a width direction of the bearing part is the same as a width direction of the clip arm.

9. The adjustable valve clip of claim 8, wherein the bearing part is of a plate-like structure.

10. The adjustable valve clip of claim 8, wherein
the bearing part is an elastic member, wherein the elastic member has a compressed state and a released state, and an area of the elastic member in the released state is greater than that of the elastic member in the compressed state; and
when the tail end of the extension arm extends beyond the end of the clip arm away from the driving section, the elastic member is in the released state.

11. The adjustable valve clip of claim 10, wherein the elastic member is a deformable mesh cage.

12. The adjustable valve clip of claim 11, wherein the mesh cage has a woven net, an end socket, and a fixing tube, wherein the end socket and the fixing tube are respectively fixed to both ends of the woven net.

13. The adjustable valve clip of claim 1, wherein the adjustable valve clip further comprises a base connecting tube, the base connecting tube is sleeved on the pushing rod and fixed with the fixing base.

14. The adjustable valve clip of claim 13, wherein a proximal end of the pushing rod defines a circular groove, the base connecting tube is provided with a resilient piece, and an end of the resilient piece is clipped into the circular groove when the clip arms are in a closing state.

15. The adjustable valve clip of claim 1, further comprising a clamping component, wherein the clamping component is configured to cooperate with the clip arm to clamp a leaflet and comprises at least two clamping arms, and each clamping arms is between the clip arm and the pushing rod.

16. The adjustable valve clip of claim 15, wherein each clamping arm has a free end and a fixing end opposite to the free end, and the fixing end of clamping arm is fixed to the fixing base.

17. The adjustable valve clip of claim 15, wherein each clamping arm is at least partially made of an elastic material with a shape memory function.

18. The adjustable valve clip of claim 15, wherein the clamping arm has a clamping surface facing the clip arm and a clamping reinforcer on the clamping surface.

19. A valve clamping system, comprising a pushing device and the adjustable valve clip of any of claims 1-18, wherein the pushing device comprises an operating handle and a pushing shaft, a proximal end of the pushing shaft is connected to the operating handle, and a distal end of the pushing shaft is detachably connected to the adjustable valve clip.

20. The valve clamping system of claim 19, wherein the pushing shaft comprises a mandrel, a bushing, an outer tube which are movably axially sleeved together, the bushing is disposed between the mandrel and the outer tube, the mandrel is detachedly connected with the pushing rod, and the mandrel is configured to drive the pushing rod to axially move.
